# EUROPEAN PATENT SPECIFICATION

(11) **EP 2 211 849 B1**
(45) Date of publication and mention of the grant of the patent: **17.07.2013**
(21) Application number: 08837957.3
(22) Date of filing: 10.10.2008
(51) Int. Cl.: A61K 31/166, A61K 31/353, A61K 31/404, A61K 31/4184, A61K 31/4412, A61K 31/506, A61K 31/517, A61K 45/06, A61P 35/00, A61P 17/00

(54) **Dexanabinol with inhibitors of BRAF or MEK for the treatment of melanoma.**
Dexanabinol mit BRAF- oder MEK-Inhibitoren zur Behandlung von Melanom.
Traitement de mélanome.

(30) Priority: 10.10.2007 GB 0719771
(43) Date of publication of application: 04.08.2010
(73) Proprietor: E-Therapeutics plc, Long Hanborough Oxfordshire OX29 8LN (GB)
(72) Inventor: YOUNG, Malcolm, Philip, Newcastle Upon Tyne NE2 4LZ (GB); THOMAS, Catherine, Mary, Newcastle Upon Tyne NE2 4LZ (GB); IDOWU, Olusola, Clement, Newcastle Upon Tyne NE2 4LZ (GB); CHARLTON, Julie Anne, Hexham NE48 2TU (GB)
(74) Representative: Gilholm, Stephen Philip
(86) International application number: PCT/GB2008/003415
(87) International publication number: WO 2009/047505

(56) References cited:
- WO-A-02/17952
- WO-A-03/077832
- GRAY-SCHOPFER VANESSA ET AL: "Melanoma biology and new targeted therapy" NATURE (LONDON), vol. 445, no. 7130, February 2007 (2007-02), pages 851-857, XP002526629 ISSN: 0028-0836
- BECKER JUERGEN C ET AL: "Molecularly targeted therapy for melanoma - Current reality and future options" CANCER, vol. 107, no. 10, November 2006 (2006-11), pages 2317-2327, XP002526630 ISSN: 0008-543X
- JUTTLER E ET AL: "The cannabinoid dexanabinol is an inhibitor of the nuclear factor-kappa B (NF-[kappa]B)" NEUROPHARMACOLOGY 200409 GB, vol. 47, no. 4, September 2004 (2004-09), pages 580-592, XP004565330 ISSN: 0028-3908
- CHANDLER L JUDSON ET AL: "Acute ethanol inhibits extracellular signal-regulated kinase, protein kinase B, and adenosine 3':5'-cyclic monophosphate response element binding protein activity in an age- and brain region-specific manner", ALCOHOLISM: CLINICAL AND EXPERIMENTAL RESEARCH, WILLIAMS AND WILKINGS, BALTIMORE, MD, US, vol. 29, no. 4, 1 April 2005 (2005-04-01), pages 672-682, XP009112366, ISSN: 0145-6008, DOI: 10.1097/01.ALC.0000158935.53360.5F
- DATABASE BIOSIS [Online] BIOSCIENCES INFORMATION SERVICE, PHILADELPHIA, PA, US 1997 BREWSTER M E ET AL: 'Clinical pharmacokinetics of escalating i.v. doses of dexanabinol (HU-211), a neuroprotectant agent, in normal volunteers' Database accession no. PREV199799759402 & BREWSTER M E ET AL: "Clinical pharmacokinetics of escalating i.v. doses of dexanabinol (HU-211), a neuroprotectant agent, in normal volunteers", INTERNATIONAL JOURNAL OF CLINICAL PHARMACOLOGY AND THERAPEUTICS, vol. 35, no. 9, 1997, pages 361-365, ISSN: 0946-1965

## Description

### Field of the Invention

The present invention provides combination medicaments and their use for the treatment of melanoma.

### Background

Incidence of melanoma cases has doubled every year since the 1940s. Melanoma is now the sixth most common cancer in men, and the seventh most common cancer in women. Its incidence is increasing in all parts of the world (Parker, *S et al,* 1997). The 5 year survival rate for melanoma is 30 to 40%, with malignant melanoma carrying the highest risk of mortality from metastasis (Jemal *et al,* 2001); Spread of the disease to distant organs such as liver, bone and brain, reduces the 5 year survival to less than 12%. There is currently no effective long-term treatment for patients with metastatic (Stage IV) melanoma. Standard chemotherapy regimens do not impart a significant long term survival benefit in these patients, and chemotherapy may be associated with a degree of morbidity due to toxicity. There is an obvious need to develop new targeted therapies for melanoma, both to prevent cancer progression and to treat advanced disease.

Finding new effective treatments for melanoma has proved very challenging. High resistance to conventional chemotherapeutic agents and radiation is a hallmark melanoma. (Smalley and Eisen, 2003; Strauss *et al.,* 2003). Research has now shifted to identifying melanoma gene mutations and the associated perturbations of signal transduction pathways, in the hope that more specific targeted therapies can be developed. Several cellular pathways important to cell proliferation, apoptosis and resistance or metastases have been shown to be activated in melanoma. Melanoma likely develops multiple defects, including loss of regulatory functions or the gain of anti-apoptotic or proliferative functions. Thus, therapeutic agents that could inhibit several signalling pathways simultaneously would be highly desirable. In addition, administration of standard chemotherapies in combination with new agents may afford the traditional chemotherapies a new lease of life in the melanoma context, if chemo resistance is inhibited. The challenge now is to develop selective agents to target these aberrant pathways.

An important signalling pathway in melanoma is the RAS/RAF mitogen-activated protein kinase (MAPK)/extracellular signal-regulated kinase (ERK) kinase (MEK)/ERK cascade. This signalling pathway leads to the phosphorylation of several cytosolic and nuclear proteins to regulate gene expression, and thus plays a critical role in cell proliferation, differentiation, senescence and survival. One of the three RAF isoforms in humans, BRAF, is mutated in 50% to 70% of melanoma cases. The ERK pathway is therefore hyperactivated in most melanoma cases. A number of small molecule inhibitors that target BRAF, or its downstream effector, MEK, are in clinical and preclinical development. Sorafenib for example, is a broad specificity kinase inhibitor that targets BRAF, CRAF and receptor tyrosine kinases. Sorafenib is 4-[4-[[4-chloro-3-(trifluoromethyl)phenyl]carbamoylamino]phenoxy]-*N*-methyl-pyridine-2-carboxamide and is described in US Patent No. 7,235,576. When used as a monotherapy, sorafenib only shows marginal clinical benefit in melanoma patients. One hypothesis put forward to explain the poor performance of sorafenib and other BRAF inhibitors clinically is the pro-survival influence of tumour necrosis factor alpha (TNF-alpha). Inflammation plays an important role in the tumour microenvironment and a number of studies have shown infiltration of TNF-alpha secreting immune cells into melanomas. Increased cancer severity has also been correlated with polymorphisms that elevate TNF-alpha expression. Combining drugs that target BRAF or MEK with an agent that inhibits the rescue pathways stimulated by TNF-alpha provides a rational approach to treating melanoma. We have previously described a novel treatment for melanoma using dexanabinol in our co-pending UK patent application No. GB0713116.2). In addition to its utility as a melanoma monotherapy, dexanabinol would be a highly suitable agent to combine with anti-BRAF or anti-MEK therapies. Not only does dexanabinol target NFKB, an important antiapoptotic and proinflammatory target in melanoma, but it also blocks the production of TNF-alpha at a post-translational level. Dexanabinol thus targets both the key proteins that act as a barrier to successful anti-BRAF and anti-MEK therapies.

Gray-Schopfer, et al "Melanoma biology and new targeted therapy" NATURE (LONDON), vol. 445, no. 7130, February 2007 (2007-02), pages 851-857; describes certain BRAF and MEK inhibitors as possible agents for the treatment of melanoma. But this document further suggests that panspecific Raf drugs would be better anti- melanoma agents than BRAF-specific drugs.

### Summary of the Invention

We have now found a method of increasing the therapeutic effectiveness of agents that target BRAF and MEK in melanoma, by combining the agents with dexanabinol. Therefore, in accordance with a first aspect, the present invention provides a pharmaceutical composition comprising dexanabinol, or a salt thereof, in combination with a second therapeutic agent that is an anti-BRAF agent selected from the group consisting of dasatinib, erlotinib, gefitinib, imatinib, lapatinib, sorafenib and sunitinib, or a salt thereof, or an anti-MEK agent selected from the group consisting of PD-325901, XL518, PD-184352, PD-318088, AZD6244 and CI-1040, and a pharmaceutically acceptable adjuvant, diluent or carrier.

The treatment of melanoma according to the invention may comprise, separately, simultaneously or sequentially inhibiting NFKB and TNF-alpha activity in a melanoma cancer cell by providing to the cell dexanabinol or a salt thereof, in combination with an anti-BRAF or anti-MEK agent as speccified in the claims.

Thus, according to a further aspect of the invention we provide dexanabinol, or a salt thereof, separately, simultaneously or sequentially in combination with a second therapeutic agent that is an anti-BRAF agent selected from the group consisting of dasatinib, erlotinib, gefitinib, imatinib, lapatinib, sorafenib and sunitinib, or a salt thereof, or an anti-MEK agent selected from the group consisting of PD-325901, XL518, PD-184352, PD-318088, AZD6244 and CI-1040, for the treatment of melanoma.

Thus, the second therapeutic agent may target BRAF. Alternatively, the second therapeutic agent may target MEK.

Examples of therapeutic agents that target BRAF include, dasatinib, erlotinib, gefitinib, imatinib, lapatinib, sorafenib and sunitinib, or a salt thereof. Thus, according to the invention the BRAF inhibitor may be selected from the group consisting of dasatinib, erlotinib hydrochloride, gefitinib, imatinib mesilate, lapatinib, sorafenib tosylate and sunitinib malate. Preferably the BRAF inhibitor is sorafenib tosylate.

Thus, according to the invention the MEK inhibitor may be selected from the group consisting of certain experimental compounds, some of which are currently in Phase 1 or Phase II studies, namely PD-325901, XL518, PD-184352, PD-318088, AZD6244 and C1-1040.

Alternatively, the treatment of melanoma may comprise the inhibition of tumorigenesis of a melanoma cancer cell by contacting the cell with an effective amount of dexanabinol, or a salt thereof, and either an anti-BRAF agent selected from the group consisting of dasatinib, erlotinib, gefitinib, imatinib, lapatinib, sorafenib and sunitinib, or a salt thereof, or anti-MEK agent selected from the group consisting of PD-325901, XL518, PD-184352, PD-318088, AZD6244 and CI-1040. Inhibition of tumorigenesis includes inducing both cytotoxicity and apoptosis in the cancer cell.

Dexanabinol, or a salt thereof, in combination with either an anti-BRAF or anti-MEK agent for the treatment of melanoma is advantageous, *inter alia,* because it shows reduced toxicity, reduced side effects and/or reduced resistance when compared to currently employed chemotherapeutic agents.

It is further contemplated that the anti-BRAF or anti-MEK agent may be administered with the dexanabinol, or a salt thereof, separately, simultaneously or sequentially.

Accordingly, and in one embodiment, the present invention provides a use of dexanabinol, and/or a salt thereof, in combination with either an anti-BRAF agent selected from the group consisting of dasatinib, erlotinib, gefitinib, imatinib, lapatinib, sorafenib and sunitinib, or a salt thereof, or anti-MEK agent selected from the group consisting of PD-325901, XL518, PD-184352, PD-318088, AZD6244 and CI-1040, in the manufacture of a medicament for the treatment of melanoma.

We further provide the use of an anti-BRAF agent selected from the group consisting of dasatinib, erlotinib, gefitinib, imatinib, lapatinib, sorafenib and sunitinib, or a salt thereof, in the manufacture of a combination therapy with dexanabinol, for the treatment or alleviation of melanoma. In addition, we provide an anti-BRAF agent selected from the group consisting of dasatinib, erlotinib, gefitinib, imatinib, lapatinib, sorafenib and sunitinib, or a salt thereof, separately, simultaneously or sequentially in combination with dexanabinol for the treatment of melanoma.

In a further aspect of the invention we provide the use of an anti-MEK agent selected from the group consisting of PD-325901, XL518, PD-184352, PD-318088, AZD6244 and CI-1040, in the manufacture of a combination therapy with dexanabinol, for the treatment or alleviation of melanoma. In addition we provide an anti-MEK agent selected from the group consisting of PD-325901, XL518, PD-184352, PD-318088, AZD6244 and CI-1040, separately, simultaneously or sequentially in combination with dexanabinol for the treatment of melanoma.

Dexanabinol and derivatives and combinations of anti-BRAF or anti-MEK agents are known *per se* and may be prepared using methods known to the person skilled in the art or may be obtained commercially. In particular, dexanabinol and methods for its preparation are disclosed in U.S. Patent No. 4,876,276.

Advantageously, in the use of the invention the compound and salts and combinations of either anti-BRAF agents or anti-MEK agents may be administered orally, or intravenously.

Thus, in the use and/or composition of the invention the compound may be put up as a tablet, capsule, dragee, suppository, suspension, solution, injection, e.g. intravenously, intramuscularly or intraperitoneally, implant, a topical, e.g. transdermal, preparation such as a gel, cream, ointment, aerosol or a polymer system, or an inhalation form, e.g. an aerosol or a powder formulation.

Compositions suitable for oral administration include tablets, capsules, dragees, liquid suspensions, solutions and syrups;
Compositions suitable for topical administration to the skin include creams, e.g. oil-in-water emulsions, water-in-oil emulsions, ointments or gels;
examples of such adjuvants, diluents or carriers are:
for tablets and dragees - fillers, e.g. lactose, starch, microcrystalline cellulose, talc and stearic acid; lubricants/glidants, e.g. magnesium stearate and colloidal silicon dioxide; disintegrants, e.g. sodium starch glycolate and sodium carboxymethylcellulose;
for capsules - pregelatinised starch or lactose;
for oral or injectable solutions or enemas - water, glycols, alcohols, glycerine, vegetable oils;
for suppositories - natural or hardened oils or waxes.

It may be possible to administer the compound and/or combination thereof or any combined regime as described above, transdermally via, for example, a transdermal delivery device or a suitable vehicle or, e.g. in an ointment base, which may be incorporated into a patch for controlled delivery. Such devices are advantageous, as they may allow a prolonged period of treatment relative to, for example, an oral or intravenous medicament.

Examples of transdermal delivery devices may include, for example, a patch, dressing, bandage or plaster adapted to release a compound or substance through the skin of a patient. A person of skill in the art would be familiar with the materials and techniques which may be used to transdermally deliver a compound or substance and exemplary transdermal delivery devices are provided by GB2185187, US3249109, US3598122, US4144317, US4262003 and US4307717.

## Claims

1. A pharmaceutical composition comprising dexanabinol, or a salt thereof, in combination with a second therapeutic agent that is an anti-BRAF agent selected from the group consisting of dasatinib, erlotinib, gefitinib, imatinib, lapatinib, sorafenib and sunitinib, or a salt thereof, or an anti-MEK agent selected from the group consisting of PD-325901, XL518, PD-184352, PD-318088, AZD6244 and CI-1040, and a pharmaceutically acceptable adjuvant, diluent or carrier.

2. A pharmaceutical composition according to claim 1 wherein the second therapeutic is an anti-BRAF agent selected from the group consisting of dasatinib, erlotinib, gefitinib, imatinib, lapatinib, sorafenib and sunitinib, or a salt thereof.

3. A pharmaceutical composition according to claim 1 wherein the anti-BRAF agent is selected from the group consisting of dasatinib, erlotinib hydrochloride, gefitinib, imatinib mesilate, lapatinib, sorafenib tosylate and sunitinib malate.

4. A pharmaceutical composition according to claim 3 wherein the second therapeutic agent is sorafenib tosylate.

5. A pharmaceutical composition according to claim 1 wherein the second therapeutic agent is an anti-MEK agent selected from the group consisting of PD-325901, XL518, PD-184352, PD-318088, AZD6244 and CI-1040.

6. Dexanabinol, or a salt thereof, separately, simultaneously or sequentially in combination with a second therapeutic agent that is an anti-BRAF agent selected from the group consisting of dasatinib, erlotinib, gefitinib, imatinib, lapatinib, sorafenib and sunitinib, or a salt thereof, or an anti-MEK agent selected from the group consisting of PD-325901, XL518, PD-184352, PD-318088, AZD6244 and CI-1040, for use in the treatment of melanoma.

7. The use of dexanabinol, or a salt thereof, in the manufacture of a combination medicament with a second therapeutic agent that is an anti-BRAF agent selected from the group consisting of dasatinib, erlotinib, gefitinib, imatinib, lapatinib, sorafenib and sunitinib, or a salt thereof, or an anti-MEK agent selected from the group consisting of PD-325901, XL518, PD-184352, PD-318088, AZD6244 and CI-1040, for use in the treatment or alleviation of melanoma.

8. The use of an anti-BRAF agent selected from the group consisting of dasatinib, erlotinib, gefitinib, imatinib, lapatinib, sorafenib and sunitinib, or a salt thereof in the manufacture of a combination medicament with dexanabinol, for the treatment or alleviation of melanoma.

9. An anti-BRAF agent selected from the group consisting of dasatinib, erlotinib, gefitinib, imatinib, lapatinib, sorafenib and sunitinib, or a salt thereof, separately, simultaneously or sequentially in combination with dexanabinol for use in the treatment of melanoma.

10. The use of an anti-MEK agent selected from the group consisting of PD-325901, XL518, PD-184352, PD-318088, AZD6244 and CI-1040 in the manufacture of a combination medicament with dexanabinol, for the treatment or alleviation of melanoma.

11. An anti-MEK agent selected from the group consisting of PD-325901, XL518, PD-184352, PD-318088, AZD6244 and CI-1040, separately, simultaneously or sequentially in combination with dexanabinol for use in the treatment of melanoma.

## Patentansprüche

1. Pharmazeutische Zusammensetzung, umfassend Dexanabinol, oder ein Salz davon, in Kombination mit einem zweiten therapeutischen Mittel, welches ein anti-BRAF-Mittel, ausgewählt aus der Gruppe, bestehend aus Dasatinib, Erlotinib, Gefitinib, Imatinib, Lapatinib, Sorafenib und Sunitinib, oder einem Salz davon, oder ein anti-MEK-Mittel, ausgewählt aus der Gruppe, bestehend aus PD-325901, XL518, PD-184352, PD-318088, AZD6244 und Cl-1040, ist, und ein pharmazeutisch annehmbares Hilfsmittel, Verdünnungsmittel oder Trägerstoff.

2. Pharmazeutische Zusammensetzung nach Anspruch 1, wobei das zweite Therapeutikum ein anti-BRAF-Mittel, ausgewählt aus der Gruppe, bestehend aus Dasatinib, Erlotinib, Gefitinib, Imatinib, Lapatinib, Sorafenib und Sunitinib, oder einem Salz davon, ist.

3. Pharmazeutische Zusammensetzung nach Anspruch 1, wobei das anti-BRAF-Mittel ausgewählt ist aus der Gruppe, bestehend aus Dasatinib, Erlotinib-Hydrochlorid, Gefitinib, Imatinib-Mesilat, Lapatinib, Sorafenib-Tosylat und Sunitinib-Malat.

4. Pharmazeutische Zusammensetzung nach Anspruch 3, wobei das zweite therapeutische Mittel Sorafenib-Tosylat ist.

5. Pharmazeutische Zusammensetzung nach Anspruch 1, wobei das zweite therapeutische Mittel ein anti-MEK-Mittel, ausgewählt aus der Gruppe, bestehend aus PD-325901, XL518, PD-184352, PD-318088, AZD6244 und CI-1040, ist.

6. Dexanabinol, oder ein Salz davon, separat, gleichzeitig oder sequenziell in Kombination mit einem zweiten therapeutischen Mittel, welches ein anti-BRAF-Mittel, ausgewählt aus der Gruppe, bestehend aus Dasatinib, Erlotinib, Gefitinib, Imatinib, Lapatinib, Sorafenib und Sunitinib, oder einem Salz davon, oder ein anti-MEK-Mittel, ausgewählt aus der Gruppe, bestehend aus PD-325901, XL518, PD-184352, PD-318088, AZD6244 und CI-1040, ist, zur Verwendung in der Behandlung von Melanom.

7. Verwendung von Dexanabinol, oder einem Salz davon, in der Herstellung eines Kombinationsmedikaments mit einem zweiten therapeutischen Mittel, welches ein anti-BRAF-Mittel, ausgewählt aus der Gruppe, bestehend aus Dasatinib, Erlotinib, Gefitinib, Imatinib, Lapatinib, Sorafenib und Sunitinib, oder einem Salz davon, oder ein anti-MEK-Mittel, ausgewählt aus der Gruppe, bestehend aus PD-325901, XL518, PD-184352, PD-318088, AZD6244 und CI-1040, ist, zur Verwendung in der Behandlung oder Verminderung von Melanom.

8. Verwendung eines anti-BRAF-Mittels, ausgewählt aus der Gruppe, bestehend aus Dasatinib, Erlotinib, Gefitinib, Imatinib, Lapatinib, Sorafenib und Sunitinib, oder einem Salz davon, in der Herstellung eines Kombinationsmedikaments mit Dexanabinol, zur Behandlung oder Verminderung von Melanom.

9. Anti-BRAF-Mittel, ausgewählt aus der Gruppe, bestehend aus Dasatinib, Erlotinib, Gefitinib, Imatinib, Lapatinib, Sorafenib und Sunitinib, oder einem Salz davon, separat, gleichzeitig oder sequenziell in Kombination mit Dexanabinol, zur Verwendung in der Behandlung von Melanom.

10. Verwendung eines anti-MEK-Mittels, ausgewählt aus der Gruppe, bestehend aus PD-325901, XL518, PD-184352, PD-318088, AZD6244 und CI-1040, in der Herstellung eines Kombinationsmedikaments mit Dexanabinol, zur Behandlung oder Verminderung von Melanom.

11. Anti-MEK-Mittel, ausgewählt aus der Gruppe, bestehend aus PD-325901, XL518, PD-184352, PD-318088, AZD6244 und CI-1040, separat, gleichzeitig oder sequenziell in Kombination mit Dexanabinol, zur Verwendung in der Behandlung von Melanom.

## Revendications

1. Composition pharmaceutique comprenant du dexanabinol, ou un sel de celui-ci, en combinaison avec un second agent thérapeutique qui est un agent anti-BRAF choisi parmi le groupe consistant en dasatinib, erlotinib, imatinib, lapatinib, sorafenib et sunitinib, ou un sel de ces derniers, ou un agent anti-MEK choisi parmi le groupe consistant en PD-325901, XL518, PD-184352, PD-318088, AZD6244 et CI-1040, et un adjuvant, diluant ou porteur, acceptable sur le plan pharmaceutique.

2. Composition pharmaceutique selon la revendication 1, **caractérisée en ce que** le second agent thérapeutique est un agent anti-BRAF, choisi parmi le groupe consistant en dasatinib, erlotinib, gefitinib, imatinib, lapatinib, sorafenib et sunitinib, ou un sel de ces derniers.

3. Composition pharmaceutique selon la revendication 1, **caractérisée en ce que** l'agent anti-BRAF est choisi parmi le groupe consistant en dasatinib, erlotinib hydrochlorure, gefitinib, mesilate de imatinib, lapatinib, sorafenib tosylate et melate de sunitinib.

4. Composition pharmaceutique selon la revendication 3, **caractérisée en ce que** le second agent thérapeutique est du tosylate de sorafenib.

5. Composition pharmaceutique selon la revendication 1, **caractérisée en ce que** le second agent thérapeutique est un agent anti-MEK choisi parmi le groupe consistant en PD-325901, XL518, PD-184352, PD-318088, AZD6244 et CI-1 040.

6. Dexanabinol, ou un sel de celui-ci, en combinaison séparément, simultanément ou séquentiellement avec un second agent thérapeutique qui est un agent anti-BRAF choisi parmi le groupe consistant en dasatinib, erlotinib, gefitinib, imatinib, lapatinib, sorafenib et sunitinib, ou un sel de ces derniers, ou un agent anti-MEK choisi parmi le groupe consistant en PD-325901, XL518, PD-184352, PD-318088, AZD6244 et CI-1040, pour utilisation dans un traitement du mélanome.

7. L'utilisation de dexanabinol, ou un sel de celui-dernier, pour la fabrication d'un médicament de combinaison avec un second agent thérapeutique qui est un agent anti-BRAF choisi parmi le groupe consistant en dasatinib, erlotinib, gefitinib, imatinib, lapatinib, sorafenib et sunitinib ou un sel de celui-ci, ou un agent anti-MEK choisi parmi le groupe consistant en PD-325901, XL518, PD-184352, PD-318088, AZD6244 et CI-1040, pour l'utilisation en vue du traitement ou le soulagement du mélanome.

8. Utilisation d'un agent anti-BRAF choisi parmi le groupe consistant en dasatinib, erlotinib, gefitinib, imatinib, lapatinib, sorafenie et sunitinib, ou un sel de celui-dernier, pour la fabrication d'un médicament de combinaison avec dexanabinol, pour le traitement ou le soulagement du mélanome.

9. Agent anti-BRAF choisi parmi le groupe consistant en en dasatinib, erlotinib, gefitinib, imatinib, lapatinib, sorafenib et sunitinib, ou un sel de celui-ci, séparément, simultanément ou séquentiellement en combinaison avec dexanabinol pour l'utilisation dans un traitement du mélanome.

10. Utilisation d'un agent anti-MEK choisi parmi le groupe consistant en PD-325901, XL518, PD-184352, PD-318088, AZD6244 et CI-1040 dans la fabrication d'un médicament de combinaison avec dexanabinol, pour le traitement ou le soulagement du mélanome.

11. Agent anti-MEK choisi parmi le groupe consistant en PD-325901, XL518, PD-184352, PD-318088, AZD6244 et CI-1040 séparément, simultanément ou séquentiellement en combinaison avec dexanabinol pour l'utilisation en vue du traitement du mélanome.
